(19) **Europäisches Patentamt / European Patent Office / Office européen des brevets**

(11) **EP 3 656 287 B1**

(12) **EUROPEAN PATENT SPECIFICATION**

(45) Date of publication and mention of the grant of the patent:
**06.03.2024 Bulletin 2024/10**

(21) Application number: **18835503.6**

(22) Date of filing: **11.07.2018**

(51) International Patent Classification (IPC):
**A61B 3/14** *(2006.01)* **A61B 3/12** *(2006.01)*

(52) Cooperative Patent Classification (CPC):
**A61B 3/12; A61B 3/14**

(86) International application number:
**PCT/JP2018/026172**

(87) International publication number:
**WO 2019/017255 (24.01.2019 Gazette 2019/04)**

(54) **OCULAR FUNDUS IMAGING SYSTEM, DISEASE ONSET RISK PREDICTION METHOD, AND OCULAR FUNDUS IMAGING METHOD**

BILDGEBUNGSSYSTEM FÜR AUGENHINTERGRUND, VERFAHREN ZUR VORHERSAGE EINES KRANKHEITSAUSBRUCHSRISIKOS UND BILDGEBUNGSVERFAHREN FÜR AUGENHINTERGRUND

SYSTÈME D'IMAGERIE DE FOND D'OEIL, PROCÉDÉ DE PRÉDICTION DE RISQUE D'APPARITION DE MALADIE ET PROCÉDÉ D'IMAGERIE DE FOND D'OEIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.07.2017 JP 2017141677**

(43) Date of publication of application:
**27.05.2020 Bulletin 2020/22**

(73) Proprietor: **Teikyo University**
**Tokyo 173-8605 (JP)**

(72) Inventors:
• **ASAYAMA Kei**
**Tokyo 173-8605 (JP)**

• **OGAWA Mitsuhiro**
**Utsunomiya-shi**
**Tochigi 320-8551 (JP)**
• **MATSUDA Hiroyuki**
**Tokyo 173-8605 (JP)**
• **STAESSEN Jan**
**2800 Mechelen (BE)**

(74) Representative: **Richly & Ritschel Patentanwälte PartG mbB**
**Sattlerweg 20**
**51429 Bergisch Gladbach (DE)**

(56) References cited:
**JP-A- 2000 041 947    JP-A- 2004 113 382**
**JP-A- 2009 125 410**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present invention relates to a fundus imaging system, and a fundus imaging method.

BACKGROUND ART

In the related art, a fundus imaging system that includes a pulse wave sensor is known (for example, refer to Patent Document 1). In the fundus imaging system described in Patent Document 1, a pulse wave of an earlobe on a side same as the eye that is imaged by a fundus imaging apparatus, is detected by a pulse wave sensor. In addition, the fundus imaging timing is determined according to the pulse wave of the earlobe. In addition, a fundus camera including the pulse wave detector is known (for example, refer to Patent Document 2). In the fundus camera disclosed in Patent Document 2, a pulse wave at the fingertip (fingertip volume pulse wave) is detected by the pulse wave detector. In addition, a xenon lamp is caused to emit light a predetermined number of times in synchronization with the peak of the pulse wave at the fingertip.

Citation List

Patent Literature

[Patent Document 1]: Japanese Unexamined Patent Application, First Publication No. 2009-125410

[Patent Document 2]: Japanese Unexamined Patent Application, First Publication No. 2013-000369

DISCLOSURE OF INVENTION

Technical Problem

Incidentally, an amount of deviation exists between a phase of a pulse wave in a fundus part and a phase of a pulse wave of the earlobe. In addition, an amount of deviation between the phase of the pulse wave of the fundus part and the phase of the pulse wave of the earlobe varies depending on a subject. Therefore, when the phase of the pulse wave of the fundus part is calculated (estimated) according to only the phase of the pulse wave of the earlobe, the calculation accuracy (estimation accuracy) of the phase of the pulse wave of the fundus part cannot be improved sufficiently. Therefore, if only the pulse wave of the earlobe is used as a pulse wave to determine an imaging timing of the fundus part as in the fundus imaging system disclosed in Patent Document 1, the imaging timing of the fundus part cannot be controlled appropriately. That is, there is a concern that the imaging timing of the fundus part controlled according to the phase of the pulse wave of the earlobe may deviate from a desired imaging timing. Similarly, an amount of deviation exists between the phase of the pulse wave of the fundus part and the phase of the pulse wave of the fingertip. The amount of deviation between the phase of the pulse wave of the fundus part and the phase of the pulse wave of the fingertip varies depending on the subject. Therefore, when the phase of the pulse wave of the fundus part is calculated according to only the phase of the pulse wave of the fingertip, the calculation accuracy of the phase of the pulse wave of the fundus part cannot be improved sufficiently. Therefore, if only the pulse wave detector for fingertip is used as the pulse wave sensor as the fundus camera disclosed in Patent Document 2, the imaging timing of the fundus part cannot be controlled appropriately. That is, when the imaging timing of the fundus part is controlled according to the phase of the pulse wave of the fingertip, there is a concern that the imaging timing may deviate from the desired imaging timing.

The present invention is to solve the above problem, and an object of the present invention is to provide a fundus imaging system, a disease onset risk prediction method, and a fundus imaging method that can appropriately control the imaging timing of the fundus part.

Solution to Problem

According to an embodiment of the present invention, a fundus imaging system includes the features defined in claim 1.

According to an embodiment of the present invention, a fundus imaging method includes the features defined in claim 2.

Advantageous Effects of Invention

According to the present invention, it is possible to provide a fundus imaging system, and a fundus imaging method that can appropriately control an imaging timing of the fundus part.

BRIEF DESCRIPTION OF DRAWINGS

FIG. 1 is a diagram showing an example of a configuration of a fundus imaging system in a first embodiment.
FIG. 2 is a diagram showing a relationship between a subject and a fundus imaging camera main body unit, a relationship between a forehead of the subject and a forehead contact unit, and a relationship between a chin of the subject and a chin reception unit at the time of imaging the fundus part of the subject.
FIG. 3 is a diagram of the forehead contact unit and the chin reception unit shown in FIG. 2 as viewed from the right side of FIG. 2.

FIG. 4A is a front view of a forehead pulse wave sensor in a state before a diffusion portion is provided.

FIG. 4B is a schematic cross-sectional view taken along line V1-V1 of FIG. 4A.

FIG. 4C is a front view of the forehead pulse wave sensor in a state after the diffusion portion is provided.

FIG. 4D is a schematic cross-sectional view taken along line V2-V2 of FIG. 4C.

FIG. 5 is a flowchart showing processing performed in the fundus imaging method by the fundus imaging system in the first embodiment.

FIG. 6 is a diagram showing an example of a configuration of a fundus imaging system according to a second embodiment.

FIG. 7 is a flowchart showing processing performed in the fundus imaging method by the fundus imaging system in the second embodiment.

FIG. 8 is a diagram showing an example of a configuration of a disease onset risk prediction system or the like in a first application example to which the fundus imaging system in the first or second embodiment is applied.

BEST MODE FOR CARRYING OUT THE INVENTION

Hereinafter, embodiments of a fundus imaging system, and a fundus imaging method in the present invention will be described with reference to the drawings.

[First Embodiment]

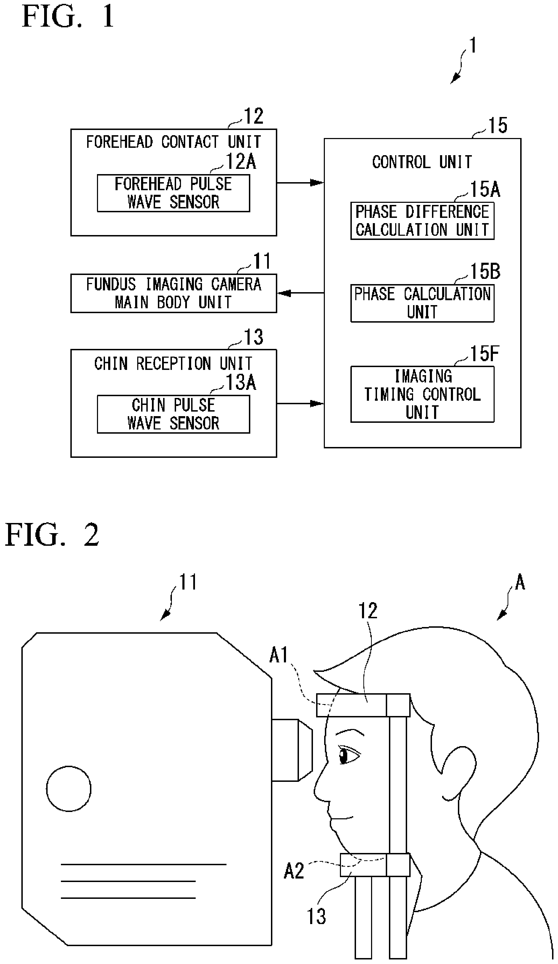

FIG. 1 is a diagram showing an example of a configuration of a fundus imaging system 1 in a first embodiment. In the example shown in FIG. 1, the fundus imaging system 1 includes a fundus imaging camera main body unit 11, a forehead contact unit 12, a chin reception unit 13, and a control unit 15. The fundus imaging camera main body unit 11 images a fundus part of a subject. The fundus imaging camera main body unit 11 is connected to the control unit 15. A signal for controlling the fundus imaging camera main body unit 11 is input to the fundus imaging camera main body unit 11 from the control unit 15.

FIG. 2 is a diagram showing a relationship between a subject A and the fundus imaging camera main body unit 11, a relationship between a forehead A1 of the subject A and the forehead contact unit 12, and a relationship between a chin A2 of the subject A and the chin reception unit 13 at the time of imaging the fundus part of the subject A. When imaging the fundus part of the subject A, the fundus imaging camera main body unit 11 images the fundus part of subject A in a state that the forehead A1 of subject A is brought into contact with the forehead contact unit 12 and that the chin A2 of the subject A is brought into contact with the chin reception unit 13.

In the example shown in FIG. 1, a forehead pulse wave sensor 12A is provided in the forehead contact unit 12. The forehead pulse wave sensor 12A detects a pulse wave of the forehead A1 of the subject A. The forehead pulse wave sensor 12A is connected to the control unit 15 and outputs a pulse wave signal of the forehead A1 of the subj ect A to the control unit 15. A chin pulse wave sensor 13A is provided in the chin reception unit 13. The chin pulse wave sensor 13A detects a pulse wave of the chin A2 of the subject A. The chin pulse wave sensor 13A is connected to the control unit 15 and outputs a pulse wave signal of the chin A2 of the subject A to the control unit 15.

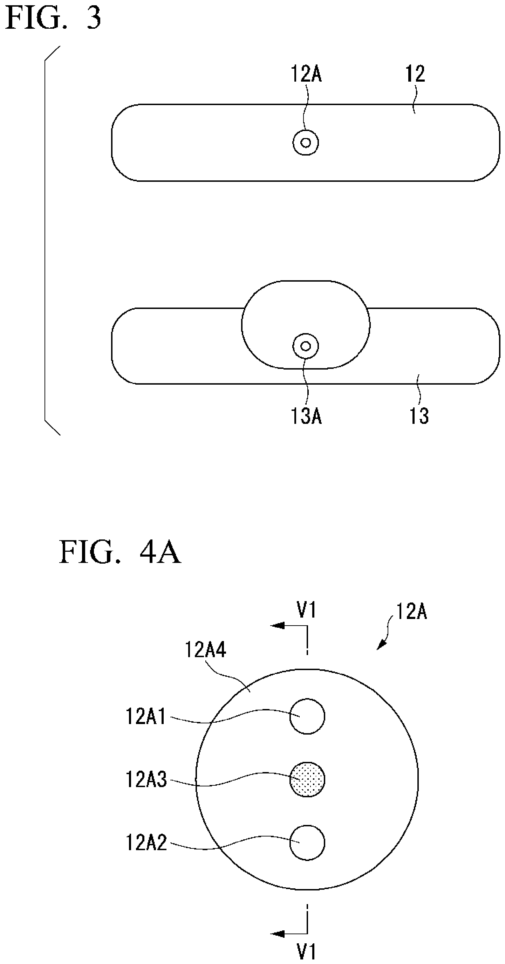

FIG. 3 is a diagram of the forehead contact unit 12 and the chin reception unit 13 shown in FIG. 2 as viewed from the right side of FIG. 2. In the example shown in FIG. 3, the forehead pulse wave sensor 12A is incorporated in the forehead contact unit 12, and the chin pulse wave sensor 13A is incorporated in the chin reception unit 13. Specifically, the forehead pulse wave sensor 12A is embedded in the forehead contact unit 12, and the chin pulse wave sensor 13A is embedded in the chin reception unit 13. For example, the same type sensors are used as the chin pulse wave sensor 13A and the forehead pulse wave sensor 12A. At the time of imaging the fundus part of the subject A, when the forehead A 1 of the subject A is brought into contact with the forehead contact unit 12, the forehead pulse wave sensor 12A comes into close contact with the forehead A1 of the subject A. In addition, at the time of imaging the fundus part of the subject A, when the chin A2 of the subject A is brought into contact with the chin reception unit 13, the chin pulse wave sensor 13A comes into close contact with the chin A2 of the subject A.

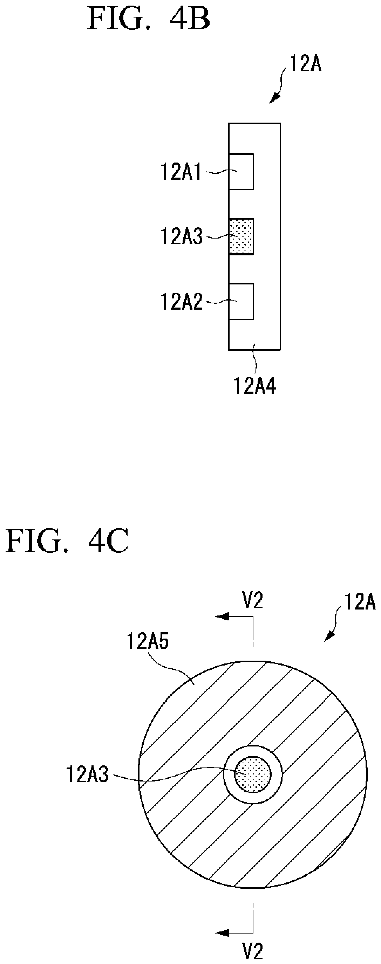

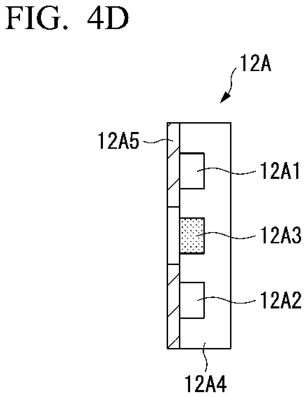

FIGs. 4A to 4D are diagrams showing examples of the configuration of the forehead pulse wave sensor 12A shown in FIG. 3. Specifically, FIG. 4A is a front view of the forehead pulse wave sensor 12A in a state before a diffusion portion 12A5 is provided. FIG. 4B is a schematic cross-sectional view taken along line V1-V1 of FIG. 4A. FIG. 4C is a front view of the forehead pulse wave sensor 12A in a state after the diffusion portion 12A5 is provided. FIG. 4D is a schematic cross-sectional view taken along line V2-V2 of FIG. 4C. In the example shown in FIGs. 4A to 4D, the forehead pulse wave sensor 12A is a reflection type. The forehead pulse wave sensor 12A includes, for example, light emitting elements 12A1 and 12A2, a light receiving element 12A3, a pulse wave sensor main body portion 12A4, and a diffusion portion 12A5. The light emitting elements 12A1 and 12A2 emit light to the subject A. The light receiving element 12A3 receives the light that is emitted from the light emitting elements 12A1 and 12A2 and reflected by the subject A. The light receiving element 12A3 outputs a pulse wave signal that is a signal according to the received light. The diffusion portion 12A5 is disposed between the light emitting elements 12A1 and 12A2, and the subject A.

In the example shown in FIGs. 4A to 4D, as shown in FIGs. 4A and 4B, the light emitting element 12A1 is disposed on the side opposite to the light emitting element 12A2 across the light receiving element 12A3. The distance between a center of the light emitting element 12A1 and a center of the light receiving element 12A3, and the distance between a center of the light receiving element 12A3 and a center of the light emitting element 12A2 are, for example, 2.54 mm, respectively. In the example shown in FIGs. 4A to 4D, the same type components are used as the light emitting elements 12A1 and 12A2. The light emitting elements 12A1 and 12A2 are green light emitting diodes (LEDs). A peak wavelength of the light emitted from the light emitting elements 12A1 and 12A2 is, for example, 520 to 570 [nm]. The light receiving element 12A3 is, for example, a photodiode or a phototransistor.

In the example shown in FIGs. 4A to 4D, as shown in FIGs. 4C and 4D, the light emitting elements 12A1 and 12A2 are covered with the diffusion portion 12A5. Therefore, the light from the light emitting elements 12A1 and 12A2 is diffused by the diffusion portion 12A5, and then, the subject A is irradiated with the diffused light. On the other hand, the light receiving element 12A3 is not covered with the diffusion portion 12A5. Therefore, the light from the subject A is received by the light receiving element 12A3 without being diffused by the diffusion portion 12A5. In the example shown in FIGs. 4A to 4D, elements having only a light emitting function are used as the light emitting elements 12A1 and 12A2, and elements having only a light receiving function are used as the light receiving element 12A3. In another example, instead, elements (for example, LEDs) that selectively have both the light emitting function and the light receiving function may be used as the light emitting elements 12A1, 12A2 and the light receiving element 12A3. In this example, whether to use the elements as the light emitting elements or as the light receiving element is appropriately selected. In addition, in this example, while using the forehead pulse wave sensor 12A, the function of the elements may be switched from the light emitting function to the light receiving function, or from the light receiving function to the light emitting function. In the example shown in FIGs. 4A to 4D, the forehead pulse wave sensor 12A includes two light emitting elements 12A1 and 12A2 and one light receiving element 12A3, however, in another example, the forehead pulse wave sensor 12Amay include one light emitting element and one light receiving element. In addition, in still another example, the forehead pulse wave sensor 12Amay include any number of light emitting elements other than two, and may include any number of light receiving elements other than one.

In the example shown in FIG. 1, the control unit 15 controls the fundus imaging camera main body unit 11. The control unit 15 includes, for example, a phase difference calculation unit 15A, a phase calculation unit 15B, and an imaging timing control unit 15F.

The phase difference calculation unit 15A calculates a phase difference (= (Lh-f - Lh-e) / v = (Tf - Te)) that is a difference between a phase of the pulse wave of the forehead A1 of the subject A and a phase of the pulse wave of the fundus part of the subject A, detected by the forehead pulse wave sensor 12A. The phase calculation unit 15B calculates phase of the pulse wave of the fundus part of the subject A according to the phase difference calculated by the phase difference calculation unit 15A and the phase of the pulse wave of the forehead A1 of the subject A detected by the forehead pulse wave sensor 12A.

If it is assumed that an effective distance of the blood vessel from a heart to the fundus part of the subject A is Lh-e, and a pulse wave propagation velocity is v, a required time from an ejection of the heart (cardiac output) to the blood vessel of the fundus part (that is, a required time for arrival of the pulse wave from the heart to the fundus part) is expressed as Lh-e / v. The pulse wave propagation velocity v is unknown because it is affected by a blood pressure and a vascular stiffness. However, the pulse wave propagation velocity v can be assumed to be constant for the same subject at the same time. The effective distance Lh-e may be estimated from the height, weight, sex, health status, and the like of the subject A.

If it is assumed that the effective distance of the blood vessel from the heart to the forehead A1 of the subject A is Lh-f, and the effective distance of the blood vessel from the heart to the chin A2 of the subject A is Lh-c, the effective distance Lh-f is expressed as the following Equation 1, and the effective distance Lh-c is expressed as following Equation 2. In Equations 1 and 2, $\alpha$ and $\beta$ are proportional constants.

$$\text{Lh-f} = \text{Lh-e} \times \alpha \ \dots \text{(Equation 1)}$$

$$\text{Lh-c} = \text{Lh-e} \times \alpha \times \beta \ \dots \text{(Equation 2)}$$

A phase difference tdiff that is a difference between the phase of the pulse wave of the forehead A1 of the subject A and the phase of the pulse wave of the chin A2 of the subject A is expressed as following Equation 3A.

$$\text{tdiff} = (\text{Lh-f} - \text{Lh-c}) / v$$

$$= (\text{Lh-e} \times \alpha - \text{Lh-e} \times \alpha \times \beta) / v$$

$$= (\text{Lh-e} \times \alpha) (1 - \beta) / v$$

$$= \text{Lh-e} \times \alpha \times (1 - \beta) / v \ \dots \text{(Equation 3A)}$$

When Equation 3A is transformed, Equation 3 below is obtained.

$$Lh\text{-}e \ / \ v = tdiff \ / \ \alpha \ / \ (1 - \beta) \ \dots \ (\text{Equation 3})$$

. In the right side of Equation 3, the phase difference tdiff is obtained from a result of detection performed by the forehead pulse wave sensor 12A and a result of detection performed by the chin pulse wave sensor 13A. The proportional constant $\alpha$ and the proportional constant $\beta$ are constants that can be obtained from data of a plurality of subjects. Therefore, the left side of Equation 3, that is, the required time (Lh-e / v) for the arrival of the pulse wave from the heart to the fundus part of the subject A can be obtained from the phase difference tdiff, the proportional constant $\alpha$, the proportional constant $\beta$, and Equation 3. However, the proportional constant $\alpha$ and the proportional constant $\beta$ may be corrected according to the height, weight, sex, health condition, and the like of subject A.

If it is assumed that a time point when the characteristic point of the pulse wave of the forehead A1 of the subject A is observed is Tf, an estimated time point Te when the characteristic point of the pulse wave appears in the fundus part of the subject A is expressed as following Equation 4 by using the relationship expressed in Equation 1 described above.

$$\begin{aligned} Te &= Tf + (Lh\text{-}e - Lh\text{-}f) \ / \ v \\ &= Tf + (Lh\text{-}e - Lh\text{-}e \times \alpha) \ / \ v \\ &= Tf + (1 - \alpha) \times Lh\text{-}e \ / \ v \ \dots \ (\text{Equation 4}) \end{aligned}$$

The imaging timing control unit 15F controls the imaging timing of the fundus part of the subject A according to the phase of the pulse wave of the fundus part of the subject A calculated by the phase calculation unit 15B.

When $\alpha < 1$, the pulse wave of the forehead A1 of the subject A precedes the pulse wave of the fundus part of the subject A. Therefore, the imaging timing control unit 15F can trigger the imaging at the same beat. That is, the imaging timing control unit 15F can control the imaging timing of the fundus part of subject A according to the characteristic point of the pulse wave of the fundus part of the subject A that is the same beat as the pulse wave of the forehead A1 of the subject A, the characteristic point of which is observed.

On the other hand, when $\alpha > 1$, the pulse wave of the fundus part of the subject A precedes the pulse wave of the forehead A1 of the subject A. Therefore, the imaging timing control unit 15F estimates a trigger timing of the next beat from the pulse wave analysis, and then, triggers the imagining. That is, the imaging timing control unit 15F controls the imaging timing of the fundus part of the subject A according to the characteristic point of the pulse wave of the fundus part of the subject A at the next beat of the pulse wave of the forehead A1 of the subject A, the characteristic point of which is observed. When the pulse fluctuates, since the trigger timing can be expected to deviate, the control unit 15 may notify of a fact that there is a high possibility of imaging failure. In this case, the imaging timing control unit 15F controls the imaging timing once again. That is, the phase difference calculation unit 15A performs the phase difference calculation again, the phase calculation unit 15B calculates the phase of the pulse wave of the fundus part of the subject A again, and the imaging timing control unit 15F controls the imaging timing of the fundus part of subject A again according to the phase.

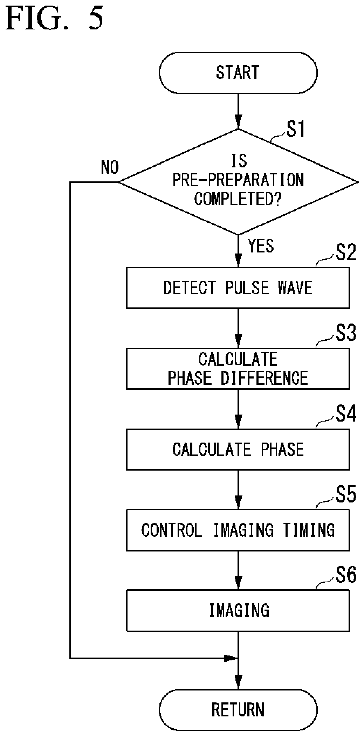

FIG. 5 is a flowchart showing processing performed in the fundus imaging method by the fundus imaging system 1 in the first embodiment. In the example shown in FIG. 5, in STEP S1, the control unit 15 determines whether or not a pre-preparation in which the forehead contact unit 12 is brought into contact with the forehead A1 of the subject A and the chin reception unit 13 is brought into contact with the chin A2 of the subject A, is completed. When the pre-preparation is completed, the process proceeds to STEP S2. On the other hand, when the pre-preparation is not completed, the routine shown in FIG. 5 is ended. In STEP S2, the forehead pulse wave sensor 12A detects the pulse wave of the forehead A1 of the subject A, and the chin pulse wave sensor 13A detects the pulse wave of the chin A2 of the subject A. Subsequently, in STEP S3, the phase difference calculation unit 15A calculates the phase difference (Tf - Te) between the phase of the pulse wave of the forehead A1 of the subject A detected by the forehead pulse wave sensor 12A and the phase of the pulse wave of the fundus part of the subject A by using the phase difference tdiff. Next, in STEP S4, the phase calculation unit 15B calculates the phase of the pulse wave of the fundus part of the subject A.

Next, in STEP S5, the imaging timing control unit 15F controls the imaging timing of the fundus part of the subject A according to the phase of the pulse wave of the fundus part of the subject A calculated in STEP S4. Next, in STEP S6, the fundus imaging camera main body unit 11 images the fundus part of the subject A at the imaging timing controlled by the imaging timing control unit 15F in STEP S5.

As described above, in the example shown in FIG. 5, the phase of the pulse wave of the fundus part of the subject A is calculated according to the pulse wave of the forehead A1 of the subject A and the pulse wave of the chin A2 of the subject A. Therefore, the calculation accuracy of the phase of the pulse wave of the fundus part of the subject A can be improved compared to a case where the phase of the pulse wave of the fundus part of the subject A is calculated according to the pulse wave of only one part of the subject A. As a result, it is possible to appropriately control the imaging timing of the fundus part of the subject A compared to the case where the phase of the pulse wave of the fundus part of the subject A is calculated according to the pulse wave of only one part of the subject A. In addition, in the example shown in FIG. 5, in order to detect the pulse wave at a part other than the forehead A1 of the subject A, the chin pulse wave sensor 13A provided in the chin reception unit 13 is used. Therefore, a pulse wave of a part other than the forehead A1 of the subject A can be obtained without complicating the pre-preparation described above.

[Second Embodiment]

The fundus imaging system 1 in a second embodiment is configured similarly to the fundus imaging system 1 in the first embodiment described above, except the points described below. Therefore, the fundus imaging system 1 in the second embodiment can achieve the effects same as the fundus imaging system 1 in the first embodiment described above, except the points described below.

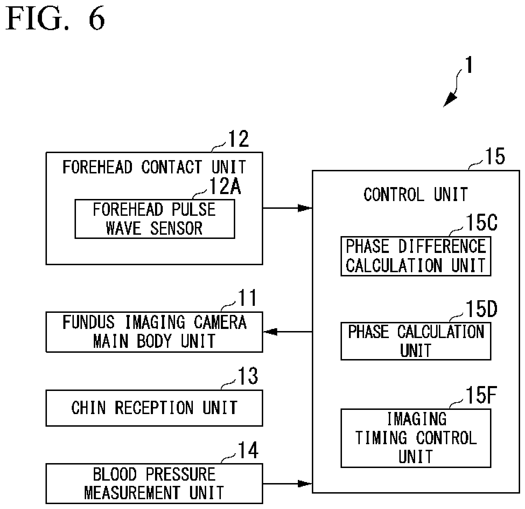

FIG. 6 is a diagram showing an example of a configuration of a fundus imaging system 1 in the second embodiment. In the example shown in FIG. 1, the chin pulse wave sensor 13A is provided in the chin reception unit 13 as described above, but in the example shown in FIG. 6, the chin pulse wave sensor is not provided in the chin reception unit 13. Instead of that, in the example shown in FIG. 6, the fundus imaging system 1 includes a blood pressure measurement unit 14. For example, the blood pressure measurement unit 14 measures a blood pressure at a finger part of the subject A, and detects a continuous blood pressure waveform (pulse wave) at the finger part of the subject A. The blood pressure measurement unit 14 is connected to the control unit 15 and outputs the pulse wave signal of the finger part of the subject A to the control unit 15.

As described above, in the example shown in FIG. 1, the control unit 15 includes the phase difference calculation unit 15A, the phase calculation unit 15B, and the imaging timing control unit 15F. On the other hand, in the example shown in FIG. 6, the control unit 15 includes a phase difference calculation unit 15C, a phase calculation unit 15D, and the imaging timing control unit 15F.

The phase difference calculation unit 15C calculates a phase difference (= (Lh-f - Lh-e) / v = (Tf - Te)) which is a difference between the phase of the pulse wave of the forehead A1 of the subject A detected by the forehead pulse wave sensor 12A and the phase of the pulse wave of the fundus part of the subject A. The phase calculation unit 15D calculates the phase of the pulse wave of the fundus part of the subject A according to the phase difference calculated by the phase difference calculation unit 15C and the phase of the pulse wave of the forehead A1 of the subject A detected by the forehead pulse wave sensor 12A.

If it is assumed that an effective distance of the blood vessel from the heart to the finger part (a measured part by the blood pressure measurement unit 14) of the subject A is Lh-fn, the effective distance Lh-fn is expressed as following Equation 5. In Equation 5, $\gamma$ is a proportional constant.

$$\mathrm{Lh\text{-}fn} = \mathrm{Lh\text{-}e} \times \alpha \times \gamma \ \dots \ (\text{Equation } 5)$$

A phase difference tdiff2, which is a difference between the phase of the pulse wave of the forehead A1 of the subject A and the phase of the pulse wave of the finger part of the subject A, is expressed as following Equation 6A.

$$\mathrm{Tdiff2} = (\mathrm{Lh\text{-}f} \cdot \mathrm{Lh\text{-}fn}) / v$$

$$= (\mathrm{Lh\text{-}e} \times \alpha - \mathrm{Lh\text{-}e} \times \alpha \times \gamma) / v$$

$$= (\mathrm{Lh\text{-}e} \times \alpha)(1 - \gamma) / v$$

$$= \mathrm{Lh\text{-}e} \times \alpha \times (1 - \gamma) / v \ \dots \ (\text{Equation } 6A)$$

When Equation 6A is transformed, Equation 6 below is obtained.

$$\mathrm{Lh\text{-}e} / v = \mathrm{tdiff2} / \alpha / (1 - \gamma) \ \dots \ (\text{Equation } 6)$$

In the right side of Equation 6, the phase difference tdiff2 is obtained from the result of detection performed by the forehead pulse wave sensor 12A and the result of detection performed by the blood pressure measurement unit 14. In addition, the proportional constant $\gamma$ is a constant obtained from data of a plurality of subjects. Therefore, the left side of Equation 6, that is, the required time (Lh-e / v) for the arrival of the pulse wave from the heart to the fundus part of the subject A can be obtained from the phase difference tdiff2, the proportional constant $\alpha$, the proportional constant $\gamma$, and Equation 6. However, the proportional constant $\gamma$ may be corrected according to the height, weight, sex, health condition, and the like of subject A.

As described above, a relationship between a time point Tf at which the characteristic point of the pulse wave of the forehead A1 of the subject A is observed and a time point Te that can be estimated that the time point Te is when the characteristic point of the pulse wave appears in the fundus part of the subject A, is expressed as Equation 4 described above.

The imaging timing control unit 15F controls the imaging timing of the fundus part of the subject A according to the phase of the pulse wave of the fundus part of the subject A calculated by the phase calculation unit 15D.

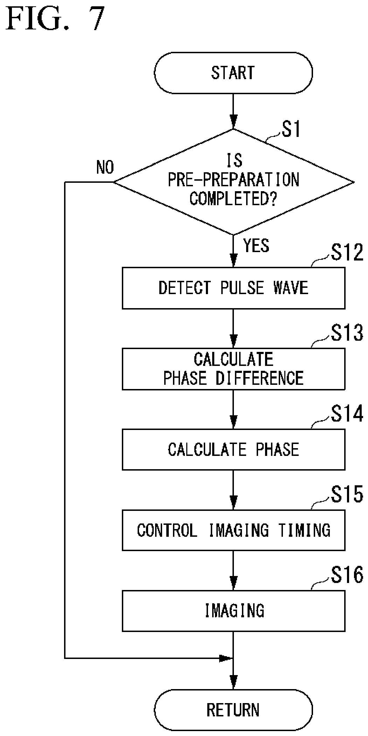

FIG. 7 is a flowchart showing processing performed in the fundus imaging method by the fundus imaging system 1 in the second embodiment. In the example shown in FIG. 7, processing similar to that in STEP S1 in FIG. 5 is performed in STEP S1. When the pre-preparation is completed, the process proceeds to STEP S12. On the other hand, when the pre-preparation is not completed, the routine shown in FIG. 7 is ended. In STEP S12, the forehead pulse wave sensor 12A detects the pulse wave of the forehead A1 of the subject A, and the blood pressure measurement unit 14 measures the blood pressure of the finger part of the subject A (that is, the blood pressure measurement unit 14 detects the pulse wave of the finger part of the subject A). Subsequently, in STEP S13, the phase difference calculation unit 15C calculates the phase difference (Tf - Te) between the phase of the pulse wave of the forehead A1 of the subject A detected by the forehead pulse wave sensor 12A and the phase of the pulse wave of the fundus part of the subject A by using the phase difference tdiff2. Next, in STEP S14, the phase calculation unit 15D calculates the phase of the pulse wave of the fundus part of the subject A.

Next, in STEP S15, the imaging timing control unit 15F controls the imaging timing of the fundus part of the subject A according to the phase of the pulse wave of the fundus part of the subject A calculated in STEP S14. Next, in STEP S16, the fundus imaging camera main body unit 11 images the fundus part of the subject A at the imaging timing controlled by the imaging timing control unit 15F in STEP S15.

As described above, in the example shown in FIG. 7, the phase of the pulse wave of the fundus part of the subject A is calculated according to the pulse wave of the forehead A1 of the subject A and the pulse wave of the finger part of the subject A. Therefore, the calculation accuracy of the phase of the pulse wave of the fundus part of the subject A can be improved compared to a case where the phase of the pulse wave of the fundus part of the subject A is calculated according to the pulse wave of only one part of the subject A. As a result, it is possible to appropriately control the imaging timing of the fundus part of the subject A compared to the case where the phase of the pulse wave of the fundus part of the subject A is calculated according to the pulse wave of only one part of the subject A. In addition, in the example shown in FIG. 7, in order to detect the pulse wave at a part other than the forehead A1 of subject A, for example, a general-purpose blood pressure measurement unit 14 is used. Therefore, a pulse wave of a part other than the forehead A1 of the subject A can be obtained without complicating the fundus imaging system 1.

[First Application Example]

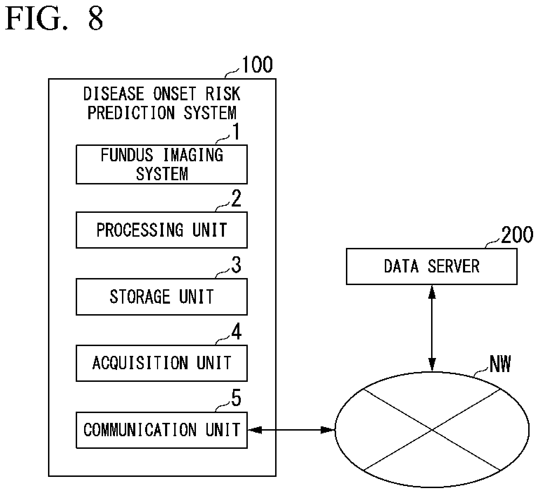

FIG. 8 is a diagram showing an example of a configuration of a disease onset risk prediction system 100 or the like in the first application example to which the fundus imaging system 1 in the first or second embodiment is applied. In the example shown in FIG. 8, the disease onset risk prediction system 100 includes, for example, the fundus imaging system 1 in the first or second embodiment, a processing unit 2, a storage unit 3, an acquisition unit 4, and a communication unit 5. For example, the processing unit 2 performs prediction or the like of an onset risk of disease to the subject A in the future. The storage unit 3 stores, for example, data or the like of the fundus part of the subject A imaged by the fundus imaging system 1. The acquisition unit 4 acquires, for example, data stored in the storage unit 3, a data server 200, or the like in response to a request from the processing unit 2. The communication unit 5 communicates with a network NW to access the data server 200, for example.

In the example in FIG. 8, at a first time point, the fundus imaging system 1 images the fundus part of the subject A at a predetermined timing in one cycle of the pulse wave of the fundus part of the subject A. The "predetermined timing" is a timing for imaging the fundus part of the subject A controlled by the above-described imaging timing control unit 15F. The data of the fundus part of the subject A imaged by the fundus imaging system 1 at the first time point is stored in the storage unit 3.

In the example shown in FIG. 8, the fundus imaging system 1 images the fundus part of the subject A at a second time point after a predetermined period has elapsed from the first time point at the same timing as the above-described predetermined timing. The data of the fundus part of the subject A imaged by the fundus imaging system 1 at the second time point is also stored in the storage unit 3. When the processing unit 2 performs the prediction of an onset risk of disease to the subject A in the future, the acquisition unit 4 acquires the data of the fundus part of the subject A at the first time point and the data of the fundus part of the subject A at the second time point that are stored in the storage unit 3, in response to the request of the processing unit 2. The processing unit 2 performs the prediction of the onset risk of disease to the subject A in the future according to changes of the fundus part of the subject A at the second time point with respect to the fundus part of the subject A at the first time point.

In the example shown in FIG. 8, the fundus imaging system 1 in the first or second embodiment is used. In the fundus imaging system 1 in the first embodiment, the phase of the pulse wave of the fundus part of the subject A is calculated with high accuracy in STEP S4 of FIG. 5, and the imaging timing of the fundus part of the subject A is appropriately controlled in STEP S5. In the fundus imaging system 1 in the second embodiment, the phase of the pulse wave of the fundus part of the subject A is calculated with high accuracy in STEP S14 of FIG. 7, and the imaging timing of the fundus part of the subject A is appropriately controlled in STEP S15. In addition, the imaging timing of the fundus part of the subject A at the first time point and the imaging timing of the fundus part of the subject A at the second time point are matched as a predetermined timing in one cycle of the pulse wave of the fundus part of the subject A. Therefore, a condition for imaging the fundus part of the subject A at the first time point can be matched with a condition for imaging the fundus part of the subject A at the second time point. In addition, in the example shown in FIG. 8, the onset risk of disease to the subject A in the future (that is, after the second time point) is predicted according to the changes of the fundus part of the subject A at the second time point with respect to the fundus part of the subject A at the first time point (that is, temporal changes of the fundus part of the subject A). Therefore, it is possible to improve a prediction accuracy compared to a case where the onset risk of disease to the subject A in the future is predicted without being based on the temporal changes of the fundus part of the subject A.

[Second Application Example]

The disease onset risk prediction system 100 in a second application example is configured similarly to the disease onset risk prediction system 100 in the first application example shown in FIG. 8. That is, the disease onset risk prediction system 100 in the second application example performs the prediction of the onset risk of disease to the subj ect A in the future in the same manner as the disease onset risk prediction system 100 in the first application example shown in FIG. 8.

In the second application example, data of the fundus part of a patient having the onset of the disease which is imaged at the same timing as the above-described predetermined timing at a third time point which is a time point before the patient has the onset of the disease, is stored in the data server 200. In addition, in the second application example, the fundus imaging system 1 images the fundus part of the subject A at the same timing as the predetermined timing described above at a fourth time point which is later than the third time point. Data of the fundus part of the subj ect A imaged by the fundus imaging system 1 at the fourth time point is stored in the storage unit 3.

In the second application example, when the processing unit 2 performs the prediction of the onset risk of disease to the subj ect A in the future, the acquisition unit 4 acquires the data of the fundus part of the patient at the third time point (that is, before the onset of disease) stored in the data server 200 and the data of the fundus part of the subject A at the fourth time point stored in the storage unit 3, in response to the request of the processing unit 2. The processing unit 2 predicts the onset risk of disease to the subject A in the future according to the fundus part of the patient before the onset of the disease imaged at the third time point and the fundus part of the subject A at the fourth time point.

In the second application example, the fundus imaging system 1 in the first or second embodiment is used. As described above, in the fundus imaging system 1 in the first or second embodiment, the phase of the pulse wave of the fundus part of the subject A is calculated with high accuracy, and the imaging timing of the fundus part of the subject A is appropriately controlled. In addition, the imaging timing of the fundus part of the subject A at the fourth time point is matched with the imaging timing of the fundus part of the patient at the third time point before the patient has the onset of the disease. Therefore, the condition for imaging the fundus part of the subject A at the fourth time point can be matched with the condition for imaging the fundus part of the patient at the third time point before the patient has the onset of the disease. In addition, in the second application example, the onset risk of disease to the subject A in the future (that is, after the fourth time point) is predicted according to the fundus part of the subject A at the fourth time point and the fundus part of the patient at the third time point before the patient has the onset of the disease (in detail, according to a matched point and a differed point between the fundus part of the subject A at the fourth time point and the fundus part of the patient at the third time point before the patient has the onset of the disease). Therefore, it is possible to improve the prediction accuracy compared to a case where the onset risk of disease to the subject A in the future is predicted without comparing the fundus part of the subject A with the fundus part of the patient before the patient has the onset of the disease.

[Third Application Example]

The disease onset risk prediction system 100 in a third application example is configured similarly to the disease onset risk prediction system 100 in the first application example shown in FIG. 8. That is, the disease onset risk prediction system 100 in the third application example performs the prediction of the onset risk of disease to the subject A in the future in the same manner as the disease onset risk prediction system 100 in the first application example shown in FIG. 8.

In the third application example, data of the fundus part of a patient having the onset of the disease which is imaged at the same timing as the above-described predetermined timing at a fifth time point which is a time point before the patient has the onset of the disease, is stored in the data server 200. In addition, data of the fundus part of the patient imaged at the same timing as the predetermined timing described above at a sixth time point which is a time point before the patient has the onset of the disease and after the fifth time point, is stored in the data server 200.

In the third application example, the fundus imaging system 1 images the fundus part of the subject A at a seventh time point at the same timing as the predetermined timing described above. Data of the fundus part of the subject A imaged by the fundus imaging system 1 at the seventh time point is stored in the storage unit 3. In addition, the fundus imaging system 1 images the fundus part of the subject A at an eighth time point after a predetermined period has elapsed from the seventh time point at the same timing as the above-described predetermined timing. The data of the fundus part of the subject A imaged by the fundus imaging system 1 at the eighth time point is also stored in the storage unit 3.

In the third application example, when the processing unit 2 performs the prediction of an onset risk of disease to the subject A in the future, the acquisition unit 4 acquires the data of the fundus part of the patient at the fifth time point (that is, before the patient has the onset of the disease) and the data of the fundus part of the patient at the sixth time point (that is, before the patient has the onset of the disease) stored in the data server 200 in response to the request of the processing unit 2. In addition, the acquisition unit 4 acquires the data of the fundus part of the subject A at the seventh time point and the data of the fundus part of the subject A at the eighth time point stored in the storage unit 3. The processing unit 2 predicts the onset risk of disease to the subject A in the future according to the changes of the fundus part of the patient imaged at the sixth time point before the patient has the onset of the disease with respect to the fundus part of the patient imaged at the fifth time point before the patient has the onset of the disease and the changes of the fundus part of the subject A imaged at the eighth time point with respect to the fundus part of the subject A imaged at the seventh time point.

In the third application example, the fundus imaging system 1 in the first or second embodiment is used. As described above, in the fundus imaging system 1 in the first or second embodiment, the phase of the pulse wave of the fundus part of the subject A is calculated with high accuracy, and the imaging timing of the fundus part of the subject A is appropriately controlled. In addition, the imaging timing of the fundus part of the subject A at the eighth time point is matched with the imaging timing of the fundus part of the subject A at the seventh time point. Therefore, the condition for imaging the fundus part of the subject A at the seventh time point can be matched with the condition for imaging the fundus part of the subject A at the eighth time point. In addition, in the third application example, the imaging timing of the fundus part of the subject A at the eighth time point is matched with the imaging timing of the fundus part of the patient at the fifth time point and the sixth time point before the patient has the onset of the disease. Therefore, the condition for imaging the fundus part of the subject A at the eighth time point can be matched with the condition for imaging the fundus part of the patient at the fifth time point and the sixth time point before the patient has the onset of the disease. In addition, in the third application example, the onset risk of disease to the subject A in the future (that is, after the eighth time point) is predicted according to the changes of the fundus part of the subject A at the eighth time point with respect to the fundus part of the subject A at the seventh time point (that is, the temporal changes of the fundus part of the subject A) and the changes of the fundus part of the patient at the sixth time point before the patient has the onset of the disease with respect to the fundus part of the patient at the fifth time point before the patient has the onset of the disease (that is, the temporal changes of the fundus part of the patient before the patient has the onset of the disease) (in detail, according to the matched point and the differed point between the temporal changes of fundus part of the subject A and the temporal changes of fundus part of the patient before the patient has the onset of the disease). Therefore, it is possible to improve the prediction accuracy compared to a case where the onset risk of disease to the subject A in the future is predicted without comparing the temporal changes of the fundus part of the subject A with the temporal changes of the fundus part of the patient before the patient has the onset of the disease.

As described above, in the present invention, in order to suppress the measurement errors when obtaining biological information, the signals itself generated by the living body is used for controlling the imaging timing, and thus, a condition of biological activity at a time of collecting the information is easily and constantly adjusted within the subject and between the subjects. Using the system in which the present invention is incorporated, the measurement accuracy of the biological information is improved, and thus, it is possible to easily improve the ability to predict diseases such as the onset of the cerebrocardiovascular disease of the subject in the future.

In a narrow sense, it is possible to easily realize the improvement in an accuracy of the images by defining measurement conditions for the fundus imaging. In the related art, when capturing the fundus image (non-mydriatic fundus image) in a state of not applying eye drops of a drug that dilates the pupil, since a flashlight is needed to be used for imaging in a dark room, it is difficult to align the imaging timing, and thus, the arteriovenous diameter is not determined by the pulsation of the blood vessel, and there is a fundamental difficulty that the measurement accuracy is lowered. In the fundus imaging system in the present invention, the fluctuation of the pulse wave of the fundus part of the subject is captured in real time from the pre-imaging stage. Because the pulse wave signal draws a waveform that goes up and down per every beat, in the fundus imaging system in the present invention, the imaging timing is controlled to a predetermined point in the waveform of the pulse wave of the fundus part of the subject or a point after a certain delay time has elapsed from that point.

As described above, imaging of the fundus using the fundus imaging system in the present invention does not differ from the imaging of the fundus in an actual and normal clinical practice to the subject. In addition, it is technically easy for a person who performs imaging to avoid the burden of attaching a new device to the subject. By using the fundus imaging system in the present invention, since the imaging timing of the fundus imaging is aligned, the measurement conditions for fundus arteriovenous diameter and endothelial thickness that increase or decrease with a pulsation can be adjusted to be constant. As a result, arteriovenous aging of the subject and changes associated with illness can be acquired with high accuracy. In addition, for the same subject, minute changes in measured values due to an intervention such as medication can be captured sharply. Furthermore, it is possible to clarify a relationship between various blood vessel information obtained from the fundus imaging and the prognosis by a long-term tracking of the subject, and thus, the blood vessel information and the condition for imaging that are useful for the prognosis prediction. Therefore, it is expected to be introduced to the next-generation diagnosis and medical cares.

The electrocardiogram indicates a contraction of a myocardium and the timing of pulse of the blood vessel to the ascending aorta, which is the origin of the systemic circulation. However, since the arrival time (pulse wave propagation time) of the blood pulse wave near the eye including the fundus varies greatly between individuals, it is not possible to estimate the pulsation state of the fundus part blood vessel with high accuracy only by synchronizing with the electrocardiogram. In particular, it is known that the pulse wave propagation time has a high positive correlation with the blood pressure. The blood pressure is affected by breathing and other factors at every heartbeat, and the pulse wave propagation time fluctuates under the influence of multiple factors other than the blood pressure. Therefore, it is difficult to control the imaging timing of the fundus part with high accuracy only from the electrocardiogram information even in the same subject.

In the related art, attempts have been made to detect diseases, such as the discovery of an infarct in the retinal artery. On the other hand, in the present invention, for example, by measuring the intima-media thickness of the fundus artery that is visualized by the fundus camera, or by adjusting the conditions of measuring an intravascular deposition of waste compounds and an microplaque formed associated therewith, and a blood turbulence flow or an arteriole blood vessel flow, it is possible to predict the onset of the cardiovascular diseases such as stroke to the subject in the future with high accuracy. That is, in the present invention, a new arteriosclerosis and cardiovascular disease risk markers can be identified that are comparable to or superior to the classic disease predictors such as the blood pressure and the blood glucose level.

Reference Signs List

1···fundus imaging system
11···fundus imaging camera main body unit
12···forehead contact unit
12A···forehead pulse wave sensor
12A1···light emitting element
12A2···light emitting element
12A3···light receiving element
12A4···pulse wave sensor main body portion
12A5···diffusion portion
13···chin reception unit
13A···chin pulse wave sensor
14···blood pressure measurement unit
15···control unit
15A···phase difference calculation unit
15B···phase calculation unit
15C···phase difference calculation unit
15D···phase calculation unit
15F···imaging timing control unit
A···subject
A1···forehead
A2···chin
100···disease onset risk prediction system
2···processing unit
3···storage unit
4···acquisition unit
5···communication unit
200···data server
NW···network

**Claims**

1. A fundus imaging system (1) comprising:

   a fundus imaging camera main body unit (11) that is configured to image a fundus part of a subject;
   a forehead contact unit (12) that is configured to be brought into contact with a forehead of the subject when imaging the fundus part;
   a chin reception unit (13) that is configured to be brought into contact with a chin of the subject when imaging the fundus part;
   a first pulse wave detection unit (12A) that is provided in the forehead contact unit (12);
   a second pulse wave detection unit (13A, 14) that is configured to detect pulse waves in a part other than the forehead of the subject; and
   a control unit (15) that is configured to control the fundus imaging camera main body unit (11), wherein the control unit (15) includes a phase calculation unit (15B) that is configured to calculate a phase of the pulse wave of the fundus part of the subject according to a phase of the pulse wave of the forehead of the subject detected by the first pulse wave detection unit and a phase of the pulse wave of the part other than the forehead of the subject detected by the second pulse wave detection unit, and an imaging timing control unit (15F) that is configured to control a timing of the imaging of the fundus part of the subject performed by the fundus imaging camera main body unit (11) according to the phase of the pulse wave of the fundus part of the subject calculated by the phase calculation unit (15B),
wherein

(a) the first pulse wave detection unit is a forehead pulse wave sensor (12A), and the second pulse wave detection unit is a chin pulse wave sensor (13A) that is provided in the chin reception unit (13) and is configured to detect the pulse wave of the chin of the subject; or
(b) the first pulse wave detection unit is a forehead pulse wave sensor (12A), and the second pulse wave detection unit is a blood pressure measurement unit (14) that is configured to measure a blood pressure of the subject.

2. A fundus imaging method comprising:

a contact step of bringing a forehead contact unit (12) into contact with a forehead of a subject and bringing a chin reception unit (13) into contact with a chin of the subject;
a detection step (S12) of detecting a pulse wave of the forehead of the subject by a first pulse wave detection unit (12A) provided in the forehead contact unit (12), and detecting a pulse wave of a part other than the forehead of the subject by a second pulse wave detection unit (13A);
a calculation step (S13) of calculating a phase of the pulse wave of the fundus part of the subject according to a phase of the pulse wave of the forehead of the subject and a phase of the pulse wave of the part other than the forehead of the subject, and
a control step (S15) of controlling a timing of the imaging of the fundus part of the subject performed by the fundus imaging camera main body unit (11) according to the phase of the pulse wave of the fundus part of the subject calculated in the calculation step, wherein

(a) the first pulse wave detection unit is a forehead pulse wave sensor (12A), and the second pulse wave detection unit is a chin pulse wave sensor that is provided in the chin reception unit (13) and is configured to detect the pulse wave of the chin of the subject; or
(b) the first pulse wave detection unit is a forehead pulse wave sensor (12A), and the second pulse wave detection unit is a blood pressure measurement unit (14) that is configured to measure a blood pressure of the subject.

**Patentansprüche**

1. Ein Fundusabbildungssystem (1), umfassend:

- eine Fundusabbildungskamera-Hauptkörpereinheit (11), die so konfiguriert ist, dass sie einen Fundusteil einer Person abbildet;
- eine Stirnkontakteinheit (12), die so konfiguriert ist, dass sie bei der Abbildung des Fundusteils mit der Stirn der Person in Kontakt gebracht wird;
- eine Kinnempfangseinheit (13), die so konfiguriert ist, dass sie mit einem Kinn der Person in Kontakt gebracht wird, wenn der Fundusteil abgebildet wird;
- eine erste Pulswellenerfassungseinheit (12A), die in der Stirnkontakteinheit (12) vorgesehen ist;
- eine zweite Pulswellenerfassungseinheit (13A, 14), die so konfiguriert ist, dass sie Pulswellen in einem anderen Teil als der Stirn der Person erfasst; und
- eine Steuereinheit (15), die zum Steuern der Fundusbildkamera-Hauptkörpereinheit (11) konfiguriert ist,
- wobei die Steuereinheit (15) eine Phasenberechnungseinheit (15B) enthält, die so konfiguriert ist, dass sie eine Phase der Pulswelle des Fundusteils der Person gemäß einer Phase der Pulswelle der Stirn der Person, die von der ersten Pulswellenerfassungseinheit erfasst wird, und einer Phase der Pulswelle des Teils, der nicht die Stirn der Person ist, der von der zweiten Pulswellenerfassungseinheit erfasst wird, berechnet
- und eine Abbildungszeitsteuereinheit (15F), die konfiguriert ist, um eine Zeitsteuerung der Abbildung des Fundusteils der Person, die durch die Fundusabbildungskamera-Hauptkörpereinheit (11) durchgeführt wird, gemäß der Phase der Pulswelle des Fundusteils der Person, die durch die Phasenberechnungseinheit (15B) berechnet wird, zu steuern,

wobei

(a) die erste Pulswellenerfassungseinheit ein Stirnpulswellensensor (12A) ist und die zweite Pulswellenerfassungseinheit ein Kinnpulswellensensor (13A) ist, der in der Kinnempfangseinheit (13) vorgesehen ist und konfiguriert ist, um die Pulswelle des Kinns der Person zu erfassen;
oder
(b) die erste Pulswellenerfassungseinheit ein Stirnpulswellensensor (12A) ist und die zweite Pulswellenerfassungseinheit eine Blutdruckmesseinheit (14) ist, die konfiguriert ist, um einen Blutdruck der Person zu messen.

2. Verfahren zur Fundusabbildung, umfassend:

- einen Kontaktschritt des In-Kontakt-Bringens einer Stirnkontakteinheit (12) mit einer Stirn einer Person und des In-Kontakt-Bringens einer Kinnempfangseinheit (13) mit einem Kinn der Person;
- einen Erfassungsschritt (S12) des Erfassens einer Pulswelle der Stirn der Person durch eine erste Pulswellenerfassungseinheit (12A), die in der Stirnkontakteinheit (12) vorgesehen ist, und des Erfassens einer Pulswelle eines anderen Teils als der Stirn der Person durch eine zweite Pulswellenerfassungseinheit (13A);
- einen Berechnungsschritt (S13) zum Berechnen einer Phase der Pulswelle des Fundusteils der Person gemäß einer Phase der Pulswelle der Stirn der Person und einer Phase der Pulswelle des anderen Teils als der Stirn der Person, und
- einen Steuerschritt (S15) des Steuerns eines Timings der Abbildung des Fundusteils der Person, der durch die Fundusabbildungskamera-Hauptkörpereinheit (11) durchgeführt wird, gemäß der Phase der Pulswelle des Fundusteils der Person, die in dem Berechnungsschritt berechnet wurde, wobei

(a) die erste Pulswellenerfassungseinheit ein Stirnpulswellensensor (12A) ist und die zweite Pulswellenerfassungseinheit ein Kinnpulswellensensor ist, der in der Kinnempfangseinheit (13) vorgesehen ist und konfiguriert ist, um die Pulswelle des Kinns der Person zu erfassen; oder
(b) die erste Pulswellenerfassungseinheit ein Stirnpulswellensensor (12A) ist und die zweite Pulswellenerfassungseinheit eine Blutdruckmesseinheit (14) ist, die konfiguriert ist, um einen Blutdruck der Person zu messen.

**Revendications**

1. Système d'imagerie de fond d'œil (1) comprenant :

une unité de corps principal de caméra d'imagerie de fond d'œil (11) qui est configurée pour imager une partie de fond d'œil d'un sujet ;
une unité de contact de front (12) qui est configurée pour être amenée en contact avec un front du sujet lors de l'imagerie de la partie de fond d'oeil ;
une unité de réception de menton (13) qui est configurée pour être amenée en contact avec un menton du sujet lors de l'imagerie de la partie de fond d'oeil ;
une première unité de détection d'onde d'impulsion (12A) qui est prévue dans l'unité de contact de front (12) ;
une seconde unité de détection d'onde d'impulsion (13A, 14) qui est configurée pour détecter des ondes d'impulsion dans une partie autre que le front du sujet ; et
une unité de commande (15) qui est configurée pour commander l'unité de corps principal de caméra d'imagerie de fond d'œil (11),
dans lequel l'unité de commande (15) comprend une unité de calcul de phase (15B) qui est configurée pour calculer une phase de l'onde d'impulsion de la partie de fond d'oeil du sujet en fonction d'une phase de l'onde d'impulsion du front du sujet détectée par la première unité de détection d'onde d'impulsion et d'une phase de l'onde d'impulsion de la partie autre que le front du sujet détectée par la seconde unité de détection d'onde d'impulsion, et une unité de commande de temporisation d'imagerie (15F) qui est configurée pour commander une temporisation de l'imagerie de la partie de fond d'œil du sujet effectuée par l'unité de corps principal de caméra d'imagerie de fond d'œil (11) en fonction de la phase de l'onde d'impulsion de la partie de fond d'oeil du sujet calculée par l'unité de calcul de phase (15B),
dans lequel

(a) la première unité de détection d'onde d'impulsion est un capteur d'onde d'impulsion de front (12A), et
la seconde unité de détection d'onde d'impulsion est un capteur d'onde d'impulsion de menton (13A) qui est prévu dans l'unité de réception de menton (13) et est configuré pour détecter l'onde d'impulsion du menton du sujet ; ou
(b) la première unité de détection d'onde d'impulsion est un capteur d'onde d'impulsion de front (12A), et

la seconde unité de détection d'onde d'impulsion est une unité de mesure de pression artérielle (14) qui est configurée pour mesurer une pression artérielle du sujet.

2. Procédé d'imagerie de fond d'œil comprenant :

une étape de contact consistant à amener une unité de contact de front (12) en contact avec un front d'un sujet et à amener une unité de réception de menton (13) en contact avec un menton du sujet ;
une étape de détection (S12) consistant à détecter une onde d'impulsion du front du sujet par une première unité de détection d'onde d'impulsion (12A) prévue dans l'unité de contact de front (12), et à détecter une onde d'impulsion d'une partie autre que le front du sujet par une seconde unité de détection d'onde d'impulsion (13A) ;

une étape de calcul (S13) consistant à calculer une phase de l'onde d'impulsion de la partie de fond d'oeil du sujet en fonction d'une phase de l'onde d'impulsion du front du sujet et d'une phase de l'onde d'impulsion de la partie autre que le front du sujet, et

une étape de commande (S15) consistant à commander une temporisation de l'imagerie de la partie de fond d'oeil du sujet effectuée par l'unité de corps principal de caméra d'imagerie de fond d'oeil (11) en fonction de la phase de l'onde d'impulsion de la partie de fond d'œil du sujet calculée à l'étape de calcul, dans lequel

(a) la première unité de détection d'onde d'impulsion est un capteur d'onde d'impulsion de front (12A), et

la seconde unité de détection d'onde d'impulsion est un capteur d'onde d'impulsion de menton qui est prévu dans l'unité de réception de menton (13) et est configuré pour détecter l'onde d'impulsion du menton du sujet ; ou

(b) la première unité de détection d'onde d'impulsion est un capteur d'onde d'impulsion de front (12A), et

la seconde unité de détection d'onde d'impulsion est une unité de mesure de pression artérielle (14) qui est configurée pour mesurer une pression artérielle du sujet.

# FIG. 1

# FIG. 2

FIG. 3

12A 12

13A 13

FIG. 4A

V1

12A

12A4

12A1

12A3

12A2

V1

# FIG. 4B

12A

12A1

12A3

12A2

12A4

# FIG. 4C

V2

12A5

12A

12A3

V2

FIG. 4D

# FIG. 5

```
        ┌─────────────┐
        │    START    │
        └──────┬──────┘
               │
               ▼              ╱S1
              ╱ ╲
       NO   ╱     ╲
    ◄──────╱   IS    ╲
           ╲ PRE-PREPARATION ╱
            ╲ COMPLETED? ╱
              ╲     ╱
               ╲ ╱
                │ YES
                ▼              ╱S2
        ┌─────────────────┐
        │ DETECT PULSE WAVE │
        └────────┬────────┘
                 │
                 ▼             ╱S3
        ┌─────────────────┐
        │    CALCULATE    │
        │ PHASE DIFFERENCE │
        └────────┬────────┘
                 │
                 ▼             ╱S4
        ┌─────────────────┐
        │ CALCULATE PHASE │
        └────────┬────────┘
                 │
                 ▼             ╱S5
        ┌─────────────────┐
        │ CONTROL IMAGING TIMING │
        └────────┬────────┘
                 │
                 ▼             ╱S6
        ┌─────────────────┐
        │     IMAGING     │
        └────────┬────────┘
                 │
                 ▼
        ┌─────────────┐
        │   RETURN    │
        └─────────────┘
```

# FIG. 6

# FIG. 7

```
        ┌─────────────┐
        │    START    │
        └──────┬──────┘
               │
               ▼          S1
          ╱─────────╲
  NO     ╱    IS     ╲
◄───────╱PRE-PREPARATION╲
         ╲ COMPLETED? ╱
          ╲─────────╱
               │ YES
               ▼          S12
        ┌─────────────┐
        │ DETECT PULSE WAVE │
        └──────┬──────┘
               │          S13
               ▼
        ┌─────────────┐
        │   CALCULATE   │
        │ PHASE DIFFERENCE │
        └──────┬──────┘
               │          S14
               ▼
        ┌─────────────┐
        │ CALCULATE PHASE │
        └──────┬──────┘
               │          S15
               ▼
        ┌─────────────┐
        │ CONTROL IMAGING TIMING │
        └──────┬──────┘
               │          S16
               ▼
        ┌─────────────┐
        │   IMAGING   │
        └──────┬──────┘
               │
               ▼
        ┌─────────────┐
        │   RETURN    │
        └─────────────┘
```

FIG. 8

DISEASE ONSET RISK
PREDICTION SYSTEM — 100

FUNDUS IMAGING
SYSTEM — 1

PROCESSING UNIT — 2

STORAGE UNIT — 3

ACQUISITION UNIT — 4

COMMUNICATION UNIT — 5

DATA SERVER — 200

NW

**EP 3 656 287 B1**

## REFERENCES CITED IN THE DESCRIPTION

### Patent documents cited in the description

- JP 2009125410 A
- JP 2013000369 A